# EUROPEAN PATENT APPLICATION

(11) **EP 1 443 107 A1**
(43) Date of publication of application: **04.08.2004**
(21) Application number: 01274634.3
(22) Date of filing: 06.11.2001
(51) Int. Cl.: C12N 15/00, C12N 5/06, C12N 5/08

(54) **PREPARING SOMATIC EMBRYO BY UTILIZING RABBIT OOCYTE**

(71) Applicant: Shanghai Second Medical University, Shanghai 200025 (CN); Sheng, Huizhen, Shanghai 200000 (CN)
(72) Inventor: SHENG, Huizhen, Shanghai 200000 (CN); CHEN, Ying, Shanghai 200000 (CN); YANG, Qingzhang, Haerbin City, Heilong jiang 150030 (CN)
(74) Representative: Catherine, Alain
(86) International application number: PCT/CN2001/001537
(87) International publication number: WO 2003/040359

(57) **Abstract**

The present invention discloses a method for preparing nuclear transfer units (nt-units, which can be also referred to as somatic embryos), which comprises transplanting mammalian cells or cell nuclei into enucleate oocytes of a species different from the nuclear donor, preferably enucleated rabbit oocytes, so as to form nuclear transfer units, and developing such nuclear transfer units to various pre-implantation stages under suitable conditions; as well as the uses of the nt-units obtained by this method.

## Description

### Field of the invention:

The present invention generally relates to cross-species nuclear transfer involving transplantation of mammalian cells or cell nuclei into enucleated oocytes of a species different from the nuclear donor. The resultant nuclear transfer units (nt-units) are cultured to reach various pre-implantation stages. These nt-units are useful for generating nuclear transfer embryonic stem cells (ntES cells). In particular, the present invention relates to the production of nt-units and the embryonic stem cells derived therefrom by transplantation of human cells or cell nuclei into enucleated animal oocytes, more preferable leporid oocytes, and most preferable rabbit enucleated oocytes.

### Background of the invention:

The method of nuclear transfer involves the transplantation of donor cells or cell nuclei into enucleated oocytes. The resultant nuclear transfer units (nt-units) are developed to various pre-implantation stages, transplanted to surrogated mothers to produce live-borne animals. The method was applied successfully to the amphibian at the end of 1950's. Briggs and King obtained the nuclear transferred frog by transferring the epithelium nuclei of enteric epithelium of the rananigromaculata into oocyte. The method of nuclear transfer was not applied to mammalian until late 1980's. It had achieved success to different extent by using various nuclear donor cells, e.g. embryo blastomere, inner cell mass, terminal embryo cell in nuclear transfer (Collas et al, Mol. Reprod. Dev., 38:264-267, 1994; Keefer et al, Biology of Reproduction, 50:935-939, 1994; Sims et al, PNAS, 90:6155-6159, 1993).

Wilmut lan et al. in Britain (Nature 385, 810-813,1997) obtained the first somatic nuclear transfer living lamb, and the donor cell thereof was derived from adult mammary gland. In 1998, the scientists in the United States completed successfully the sequential nuclear transplantation of mouse somatic cell nuclei (Wakayama, et al. Nature 394: 369-374,1998). in 1999, scientists obtained mouse nuclear transfer embryonic stem cells (ntES cells) from mouse nt-units at the blastocyst stage (Teruhiko, et al. PNAS 96:14984-14989, 1999). The success of nuclear transplantation techniques using adult somatic cells is not only a progress in techniques but also a progress in understanding of development. The fact shows that it is possible for highly differentiated adult somatic cell nuclei to form new individuals by reprogramming the somatic cell nuclei and lead them reenter development.

Cross-species nuclear transplantation techniques involve the transplantation of donor cells or cell nuclei into enucleated oocytes of a species different from the nuclear donor. The resultant nt-units are cultured to obtain blastocysts. In the case of animal cloning, nt-units at the blastocyst stage are implanted to the uterus to obtain live-borne animals. In therapeutic cloning, when human somatic cell nuclei are used as nuclear donor, nt-units at the blastocyst stage are dissociated to obtain embryonic stem cells.

Tanja Dominko, et al. (Biology of Reproduction, 60 (6), 1496-502, 1999) transplanted somatic cell nuclei of various mammalian species (e.g. cows, sheep, pigs, monkeys, and rat), into enucleated bovine oocytes to form nt-units. Blastocysts were obtained from these cross-species nt-units. This experiment shows that nuclear transfer units can be obtained by transplantation of donor cells or cell nuclei into enucleated oocytes of a species different from the nuclear donor. The nt-units resulting from cross-species nuclear transfer have the potential to develop to the blastocyst at least. The fact that oocytes of one mammalian species can reprogram effectively somatic nuclei of another mammalian species demonstrates that mechanisms controlling reprogramming are largely conserved in mammals.

For human medicine, cross-species somatic cell nuclear transfer techniques may be useful in therapeutic cloning in which a large quantity of oocytes is needed to reprogram human somatic cell nuclei. Because the human being's oocyte is very scarce, it can hardly meet the needs of clinical and laboratory use, and therefore it would be highly necessary to find other source of oocytes to satisfy the needs for both experimental and clinical applications.

As described above, the bovine oocytes are routinely used in the nuclear transfer techniques between different species. The mature oocyte can be obtained from the ovaries or reproductive tract of a cow directly by irrigation technique. However, as the techniques are very complicated, the output is unfertile (8-10 cells), and it is needed to raise a lot of bovine, the cost would be very high. (8-10 oocytes can be obtained from each bovine, which is injected artificial hormone in 5 days for promoting ovulation during the periods of the nature estrus of bovine of 21 days.) Thus, It is very expensive to obtain mature oocytes from living cows.

A readily available source of bovine oocytes is slaughterhouse's materials, but the oocytes available are often from either the older, weakling, ill, handicapped animals or animals polluted by pathogens e.g. that cause tuberculosis, foot and mouth disease; i.e., the quality of oocytes is often unstable. Furthermore, the cows in the slaughterhouse usually come from varied areas, and therefore their genetic background is usually unclear. On the other hand, the obtained oocytes could only be used after maturing by being cultured in vitro for 18 to 24 hours. Moreover, the in vitro maturated oocytes are often not as good as the in vivo maturated oocyte.

### Objects of the invention

The present inventors discovered that nuclear transfer units (nt-units) can be obtained by transplantation of human cells, e.g. adult human somatic cells or cell nuclei, into enucleated animal oocytes. The nt-units resulted from cross-species nuclear transfer have the potential to develop to the blastocyst at least. The result was the first proof that human somatic nuclei can be reprogrammed after being transplanted into enucleated oocytes and develop to the blastocyst stage. These results further demonstrated the feasibility of cross-species nuclear transplantation, e.g. the transplantation of human cells or cell nuclei into enucleated oocytes of a leporid animal, e.g. a rabbit, to produce nt-units capable of development to the blastocyst stage.

Therefore, it is an object of the invention to provide an improved method for producing nt-units by cross-species nuclear transfer.

It is another object of the invention to provide a method for producing nt-units, involving the transplantation of nuclei of human or animal cells or cell nuclei into enucleated oocytes of a species different from the nuclear donor.

It is a more specific object of the invention to provide a method involving transplantation of the nuclei of human cells into enucleated oocytes of a non-human mammalian species.

It is another specific object of the invention to provide a novel method involving transplantation of human cells or cell nuclei into enucleated animal oocytes, e.g. rabbit enucleated oocytes.

It is another object of the invention to provide a method involving transplantation of human nuclei, e.g. adult somatic nuclei, into enucleated human oocytes.

With the foregoing and other objects, advantages and features of the invention that will become hereinafter apparent, the nature of the invention may be more clearly understood by reference to the following detailed description of the preferred embodiments of the invention and to the appended claims.

### Briefs description of the figures

Figure 1 is a photograph of the fibroblasts obtained from human foreskin.
Figure 2 is a photograph of the rabbit oocytes.
Figure 3 is a photograph of the nt-unit (obtained by injection of the somatic cell into zona pellucid) at the 4-cell stage.
Figure 4 is a photograph of the nt-unit (obtained by injection of the somatic cell into zona pellucid) at the morula stage.
Figure 5 is a photograph of the nt-unit (obtained by injection of the somatic cell into zona pellucid) at the blastocyst stage.
Figure 6 is a photograph of the nt-unit (obtained by injection of the somatic cell into zona pellucid) at the hatching blastocyst stage.
Figure 7 is a photograph of the nt-unit (obtained by injection of the somatic cell into the cytoplasm of the oocyte) at 4-cell stage.
Figure 8 is a photograph of the nt-unit (obtained by injection of the somatic cell into the cytoplasm of the oocyte) at the morula stage.
Figure 9 is a photograph of the nt-unit (obtained by injection of the somatic cell into the cytoplasm of the oocyte) at the blastocyst stage.
Figure 10 is a photograph of the nt-unit (obtained by injection of the somatic cell into the cytoplasm of the oocyte) at the hatching blastocyst stage.
Figure 11 is a table showing somatic cells from donors at different ages formed blastocysts with comparable efficiency.
Figure 12 is a photograph of an ntES cell colony derived from a human somatic cell reprogrammed by a rabbit oocyte.
Figure 13 is a photograph showing a normal human karyotype of an ntES cell at the 26^{th} passage.

### Detailed description of the invention

In the subject invention, nuclear transfer or nuclear transplantation is used interchangeably.

In the subject invention, nuclear transfer units (nt-units) and somatic embryos are used interchangeably.

In the subject invention, nuclear transfer embryonic stem cell (ntES cell) or somatic cell derived embryonic stem cell (S-ES) are used interchangeably.

The term "nuclear transplantation" referred herein means the transplantation of donor cells or cell nuclei into enucleated oocytes. The resultant nt-units are cultured to various pre-implantation stages (e.g. the blastocyst stage) and are allowed, in the case of animal cloning, to further develop into live-bone animals. For nuclear transfer, somatic cells or cell nuclei of various species, for example, primates, ungulates, amphibians, rodents species, can all be used as nuclear donors. Human oocytes may be used in nuclear transplantation, as well as oocytes from other species, including those derived from primates, ungulates, amphibians, rodents, etc.

The term "homogeneous nuclear transplantation techniques" herein involves transplantation of donor cells or cell nuclei into enucleated oocytes from the same species. The term "cross-species nuclear transplantation techniques" herein involves transplantation of donor cells or cell nuclei into enucleated oocytes of a species different from the nuclear donor.

The term "nuclear transfer unit (nt-unit)" herein refers to a unit derived from the combination of a nuclear donor and an enucleated oocyte. The nuclear donor and the enucleated oocyte may be from the same or different species. The resultant nt-unit has the potential to develop to all the pre-implantation stages, and is named accordingly. For example, a nt-unit that has developed to the blastocyst stage is referred to as a nt-unit at the blastocyst stage.

In a preferred embodiment, an appropriate small animal, e.g. rabbit, is used as the oocyte donor in nuclear transfer. By feeding these animals with the standard food in case, it would be more easily to administrate and control disease, and thus the animals can be made SPF (specific pathogen free), resulting in a lower cost. About 30 oocytes can be routinely obtained from each superovulated rabbit, which has been injected artificial hormone in 4 days for promoting ovulation during the period of the nature estrus of rabbit of 7-9 days.

The present inventors discovered that the nt-unit obtained by transplantation of a human cell or cell nucleus, specifically a human fibroblast into an enucleated rabbit oocyte can produce a nt-unit with the potential to develop to the blastocyst stage. Based on the fact that human cell nuclei can be effectively reprogrammed by the rabbit oocytes, it is reasonable to expect that human somatic cells may be transplanted into oocytes of other non-rabbit mammalian species, e.g. primates, ungulates, and other rodents, to produce nuclear transfer units. Further, using similar methods, it is also possible to transfer human cells or nuclei into human oocytes to produce nt-units at various pre-implantation stages, e.g. the blastocyst stage.

Therefore, in its broadest sense, the present invention involves transplanting mammalian (including human) cells or cell nuclei into enucleated oocytes of a species different from the nuclear donor to produce nt-units at various pre-implantation stages. For example, the invention may involve the transplantation of a human cell or cell nucleus into an enucleated oocyte of a non-human mammalian species to produce a nt-units at the blastocyst stage. The resultant nt-unit may be used to obtain embryonic stem cells, or embryonic stem-like cells, or other types of embryo-derived stem cells for clinical therapies.

The approach of therapeutic cloning involves transplantation of a patient's somatic cells into enucleated mammalian oocytes to produce nt-units at various pre-implantation stages for isolation of ntES cells. The resulted ntES cells have the same genotype as that of the patient, and will most likely be recognized by the immune system of the patient as "self", thus will not be rejected by the patients immune. Therapeutic cloning provides an approach to solve the immune rejection problem commonly observed in transplantation medicine.

ntES cells derived from therapeutic cloning can be used to treat diseases. Therefore, the non-human oocytes used to reprogram human somatic nuclei must meet the following requirements. First, the oocytes must not carry any pathogens which can infect the human being involved, e.g. bacteria, virus, mycoplasma etc. Second, they must be able to reprogram effectively human nuclei and to allow the development of nt-units into blastocyst or hatching blastocyst. Furthermore, their hereditary background must be clear and the quality is stabile, and can be obtained in sufficient quantity.

Compared with the oocytes of bovine and sheep, the rabbit oocytes are of several advantages. They are more readily available, in lower cost, in more stabile quality, with clearer genetic background, and require no additional procedure for maturation in vitro before nuclear transplantation. In a special embodiment, the present invention provides a novel method for producing nt-units by cross-species nuclear transplantation that involves transplanting a human cell or cell nucleus into an enucleated rabbit oocyte to produce a nt-unit, which may be used to obtain embryonic stem cells or embryonic stem-like cells or other types of embryo-derived stem cells. As described above, people only used large ungulate animal cells (involving bovine and sheep oocytes) as the oocyte donor. Compared with bovine and sheep oocytes, the rabbit oocytes have the advantages of lower cost, more readily available and being produced in large quantity. Most important, it is possible to obtain rabbits and rabbit oocytes from the animals of SPF (special pathogen free) grade, pathogen-free oocytes are ultimately important for obtaining pathogen-free ntES cells, it is critical for therapeutic cloning when the resultant stem cells will be used to treat human diseases.

The present inventors also discovered that the rabbit oocytes could effectively reprogram human cell nuclei to produce blastocysts. Therefore, rabbit oocytes could be a preferable replacement of human oocytes to bovine and sheep in research and in therapeutic cloning.

Thus, in a preferred embodiment, the invention comprises the production of human nt-units at various pre-implantation stages by transplantation of human cells or cell nuclei into enucleated animal oocytes, more preferable leporid oocytes, and most preferable rabbit enucleated oocytes.

In general, nt-units will be produced by interspecies nuclear transfer process comprising the following steps:
(i) obtaining desired mammalian cell or cell nuclei as the nuclear donor;
(ii) obtaining oocytes from a suitable source, e.g. a mammal and more preferably a leporid, e.g. rabbit;
(iii) enucleating the above oocytes;
(iv) transferring mammalian cells or cell nuclei into enucleated oocytes and activating the nt-units; and
(v) culturing the resultant nt-units to obtain blastocysts or hatching blastocysts.

### Nuclear transfer technique

Nuclear transfer technique or nuclear transplantation techniques are known in the papers and were described in many of the references cited in the Background of the Invention. See, in particular, Wilmut, et al, Nature, 385:810-813, 1997; Campbell, et al, Biology of Reproduction, 49 (5): 933-942, 1993; Collas, et al, Mol. Reprod. Dev, 38:264-267, 1994; Keefer, et al. Biology of Reproduction, 50:935-939, 1994; Sims, et al. PNAS, 90:6155-6159, 1993; also, Patent WO 94/26884, WO 94/24274; and WO 90/03432, which are incorporated by reference in their entirety herein.

### The nuclear donor

In the invention, the cells used as nuclear donor were derived from human cells, preferable human fibroblast.

Human or animal cells, preferably mammalian cells, may be obtained and cultured as described in the art. Human and animal cells useful in the present invention include, by way of examples, epithelial cells, neural cells, epidermal cells, keratinocytes, hematopoietic cells, melanocytes, chondrocytes, nucleated erythrocytes, macrophages, mononuclear cells, fibroblasts, cardiac muscle cells, and other muscle cells, etc. Moreover, the human cells used for nuclear transfer may be obtained from different organs, e.g. skin, lung, pancreas, liver, stomach, intestine, heart, reproductive organs, bladder, kidney, urethra and other urinary organs, etc. These are just examples of suitable donor cells. Suitable donor cells, i.e., cells useful in the subject invention, may be obtained from any cell or organ of the body. This includes all somatic cells.

### Oocytes

The oocytes used for nuclear transfer may be obtained from various animals including mammals and amphibians. Suitable mammalian sources for oocytes include sheep, bovine, pigs, horses, rabbits, guinea pigs, mice, hamsters, rats, primates, etc. In the preferred embodiments, the oocytes will be obtained from leporid, e.g. a rabbit.

It has been discovered that the mature metaphase II stage oocyte could be collected surgically from the reproductive tract from either non-superovulated or superovulated rabbits 14 to 24 hours after onset of estrus or after the injection of human chorionic gonadotropin (hCG) or a similar hormone, preferable after 15 to 18 hours.

Methods for isolation of oocytes are as well described in the art. Essentially, the method,will comprise isolating oocytes from the ovaries or reproductive tract of a mammal or amphibian, e.g. a rabbit.

The degree of maturation of the oocyte in nuclear transfer has been reported to be a significant factor in the success of nuclear transfer methods (See Prather et al., Differentiation, 48,1-8,1991). In general, previous successful mammalian embryo cloning used the metaphase II stage oocyte as the recipient oocyte. Because it is believed that the oocyte at this stage can be effectively "activated" to reprogram the introduced nucleus in a similar way as a fertilizing sperm, and to produce nt-units.

### Enucleation

It has been discovered in the present invention that the mature oocytes obtained from the New Zealand rabbit should be enucleated 14 to 24 hours after the injection of human chorionic gonadotropin (hCG). Before enucleation, the oocytes will be placed in M2 culture medium (Sigma) containing hyaluronidase prior to the removal of cumulus cells. This may be affected by repeated pipetting through pipettes with very small inner diameter or by vortexing briefly. The stripped oocytes are then screened for those containing polar bodies, and the selected metaphase II oocytes has, as confirmed by the presence of polar bodies, are then used for nuclear transfer and enucleation.

Generally, the collected immature oocytes from animal ovaries should be matured in vitro as desired, until the oocytes are in the metaphase II stage.

For the New Zealand rabbit, enucleation preferably should be performed not more than 20 hours after the injection of human chorionic gonadotropin (hCG), and more preferably 16 to 18 hours.

Enucleation may be accomplished microsurgically using a micropipette to remove the polar body and the adjacent cytoplasm. The efficiency of enucleation may be examined by staining the removed polar body and chromatin with Hoechst 33342 dye, and observed DNA under ultraviolet irradiation rapidly.

### Preparation of nt-units

According to the methods well known in the art, nt-unit can be prepared, e.g. by injection into the zona pellucid and by injection into the cytoplasm.

### Injection into the zona pellucid:

A single animal or human somatic cell or cell nucleus will be transferred into the perivitelline space of the enucleated oocyte. The membranes of the somatic cell rabbit oocyte is electrofused in a 0.5 mm chamber by application of an electrical pulse of 90-120V for about 60 µ sec for 1-2 times or frequently in the electrofusion media, e.g., mannitol, and sucrose, after injection of human chorionic gonadotropin (hCG) for 16 to 20 hours. Typical activation will be done in a shorter time after electrofusion, in general, less than 24 hours, preferable 4-9 hours. After fusion, the resultant nt-units are then placed in a suitable medium, e.g. RD, M199, DMEM culture medium.

Electrofusion is accomplished by providing a pulse of electricity that is sufficient to cause a transient break down of the plasma membrane. This breakdown process of the plasma membrane is very short because the membrane reformed rapidly. Essentially, if two adjacent membranes are induced to break down, upon reformation, the lipid bilayers intermingle and small channels will open between the two cells. Due to the thermodynamic instability of such a small opening, it enlarges until the two cells fuse into one. Reference is made to U.S. Patent 4,997,384 by Prather et al., (incorporated by reference in its entirety herein), which provides a further discussion of this process. A variety of electrofusion media can be used including, e.g. sucrose, mannitol, sorbitol and phosphare buffered solution. Fusion can be also accomplished by using Sendai virus as a fusogenic agent.

The nt-unit may be activated by known methods. Such methods include, e.g. culturing the nt-unit at sub-physiological temperature, essentially by applying a cold, or actually a low temperature shock to the nt-unit. This may be most conveniently done by culturing the nt-units at room temperature, which is low relative to the physiological temperature.

Suitable oocyte activation methods are the subject of U.S. Patent No. 5496720 to Susko-Parrish et al., which is herein incorporated by reference.

For example, oocyte activation may be effected by sequentially:
(i) increasing levels of divalent cations in the oocyte, and
(ii) reducing phosphorylation of cellular proteins in the oocyte.

The methods of increasing divalent cation levels will generally be affected by e.g. the addition of kinase inhibitors, e.g. serine-threonine kinase inhibitors, such as 6-dimethyl-amino-purine, staurosporine, 2-aminopurine, and sphingosine.

Alternatively, phosphorylation of cellular proteins may be inhibited by the introduction of a phosphatase into the oocyte, e.g. phosphatase 2A and phosphatase 2B.

### Injection into the cytoplasm:

Also, other methods may be used in nuclear transplantation, e.g. injection the nucleus directly into the oocyte cytoplasm rather than using electroporation fusion (Collas and Barnes, Mol. Reprod. Dev., 38: 264-267 1994).

### Culture of nt-units

Activated nt-units may be cultured in vitro to obtain blastocysts. In the following examples that are the preferred embodiments of the present invention, the highest efficiency on culturing blastocyst is achieved when culturing the nt-units in the RD culture medium (see the details of the example).

For example, activated nt-units may be transferred to microdrops culture medium consisting of 50 microliters of tissue culture medium, e.g. RD, M199, DMEM, etc, under a layer of paraffin at 38°C and 5% CO₂.

According to the inventor's experience, for human somatic cell/enucleated rabbit oocyte derived nt-units, the blastocyst will be obtained about 6-7 days after activation of the nt-units. The nt-units obtained through cross-species nuclear transfer will typically exhibit an appearance and cellular characters similar to the nuclear donor species rather than the oocyte donor species. For example, nt-units formed between human somatic nuclei and enucleated rabbit oocytes exhibit a development schedule similar to human embryo, which forms the blastocyst in about 6-7 days, rather than that of the rabbit embryo, which forms the blastocyst in about 3-4 days in culture.

The media for culture and maintenance of the rabbit embryo are well documented in the literature, e.g. DMEM+15% FCS; M199+15% FCS; RD+15% FCS, etc. Any of the above may also involve co-culture with a variety of cell types such as granulose cells, oviduct cells, uterine cells, and STO cells

In the present invention, the resulted nt-units may be used to derive human embryonic stem cells or embryonic stem-like cells or any other types of embryo-derived stem cells, which have applications in the medical field and will provide a new method in the treatment of numerous genetic diseases.

In the practical applications, the nuclear transplantation techniques may be used in saving the species close to extinction, e.g. panda. In these species, larger number of female oocytes is hardly to obtain as the number of female oocytes is rare. Transplant their nuclei as donor cells into different species enucleated oocytes. The resultant nt-units are cultured in vitro to obtain the blastocyst and transplanted into the pregnant-mother to develop a normal individual.

In order to more clearly describe the subject invention, the following examples are provided.

### Example

### Example 1

### The preparation of nuclear donor cells for nuclear transfer

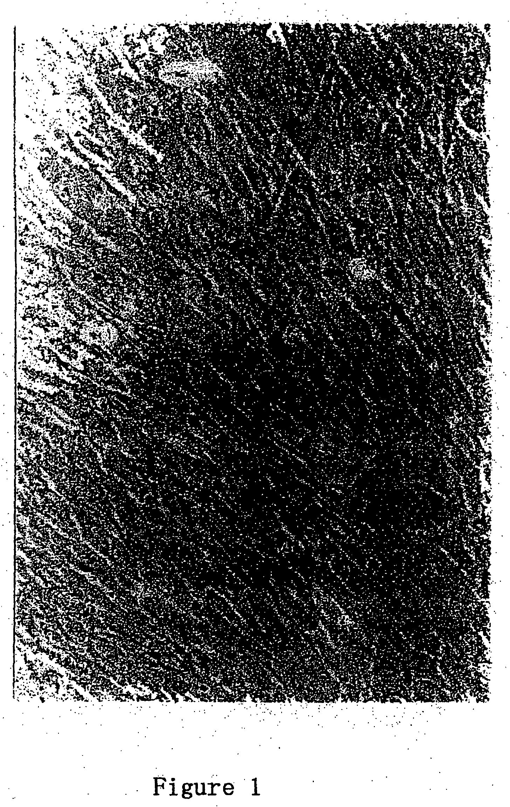

Foreskin tissue obtained from surgery with informed consent was minced and washed with PBS, centrifuged at 1000 rpm for 5 minutes, and discarded the supernatant. The tissue minced was digested by 0.05% Trypsin/0.02% EDTA (Gibco) at 37°C for 30 minutes. Remove cell suspension and centrifuge at 1000 rpm for 5 minutes. Discard the supernatant and culture the cell pellet in 90% DMEM (Gibco) + 10% FBS (Hyclone)+50 IU/ml penicillin-streptomycin (Gibco). Re-suspend in plate and incubate at 37°C, 5% CO₂, with the medium changed every 3 days. Passage after the cell grows to confluent and the cells of the 7^{th}-20^{th} passage are used as nuclear donor cells (Fig. 1).

### Oocyte preperation

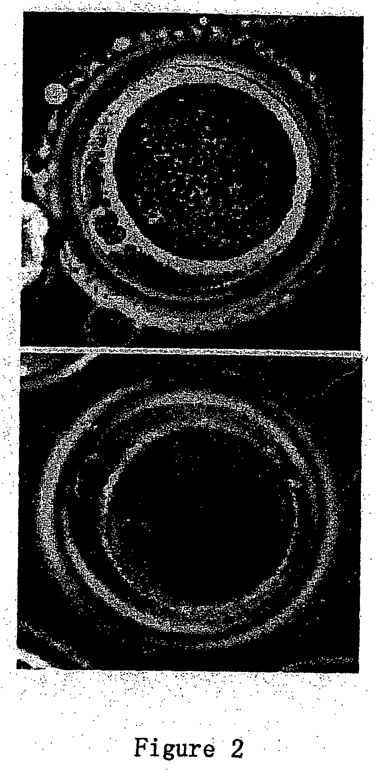

Mature New Zealand female Rabbit, 2.5-3kg, was superovulated with a single dose injection of 100 IU PMSG (i.m.) (The first bio-pharmaceutical company, Shanghai), followed by a single dose injection of 100 IU hCG (i.v.) 72 hours later. Mature oocytes were flushed out of the oviducts 14-16 hours after hCG injection using pre-gassed M2 solution (Sigma). Oocytes were put into a solution containing 300 IU/ml hyaluronidase to remove cumulus cells. Observe under anatomical lens and pick out the oocytes with a glass needle after the particles are dispersed. The oocytes (Fig. 2) were then washed in M2 solution for 3 times.

### Nuclear transfer procedures

### Injection into the zona pellucid:

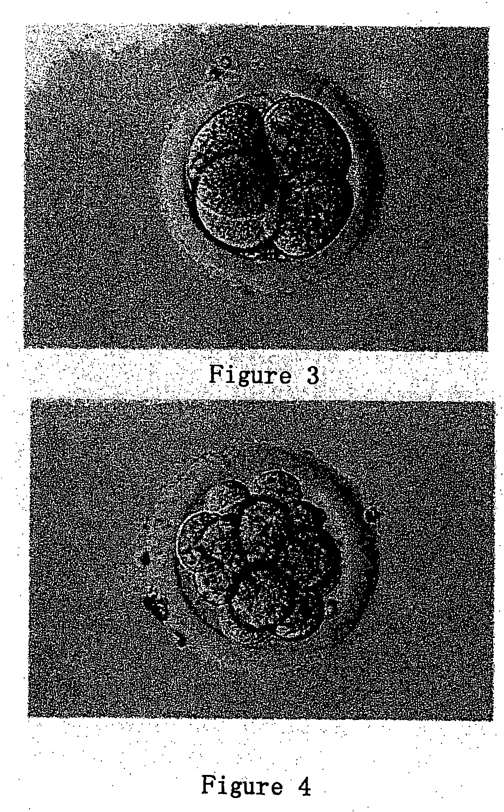

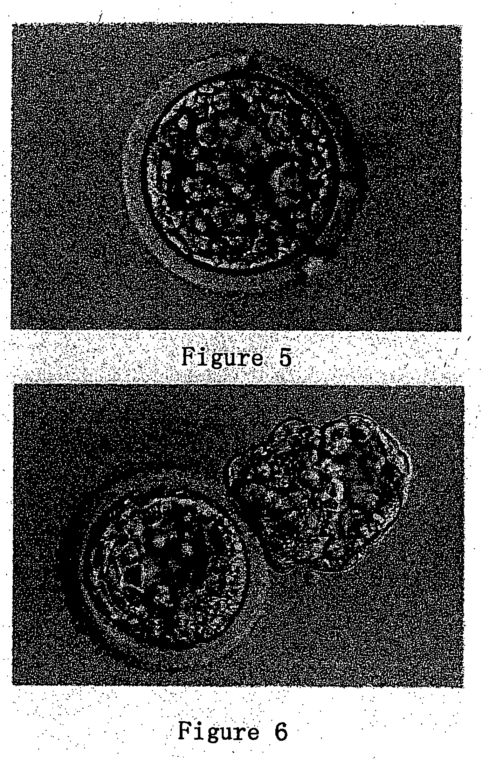

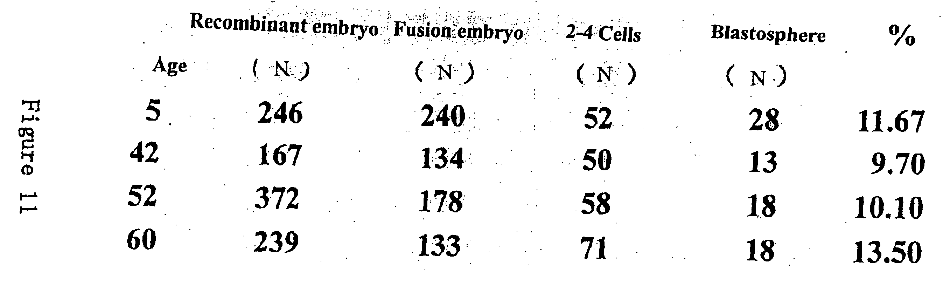

Oocytes were manipulated in M2 medium with 7.5 µg/ml Cytochalasin B (Sigma) and incubated and enucleated by a needle with a bevel after maintained for 10 minutes at room temperature. After that, put the single donor fibroblast into the perivitelline space of enucleated rabbit oocyte, forming nt-units. The nt-units were equilibrated in a fusion buffer solution containing 0.3 M Glucose (Sigma); 0.1 mM MgCl₂ (Sigma); 0.05 mM CaCl₂ (Sigma) and stimulated with a single direct current pulse of HV 120V for 60 µseconds. After stimulation, the nt-units were incubated in RD medium, consisting of DMEM 42.5% (Gibco), RPMI-1640 42.5% (Gibco), and 15% FBS (Hyclone). After 6-7 days, blastocysts were obtained (Figs. 3-6,11).

### Injection into the cytoplasm

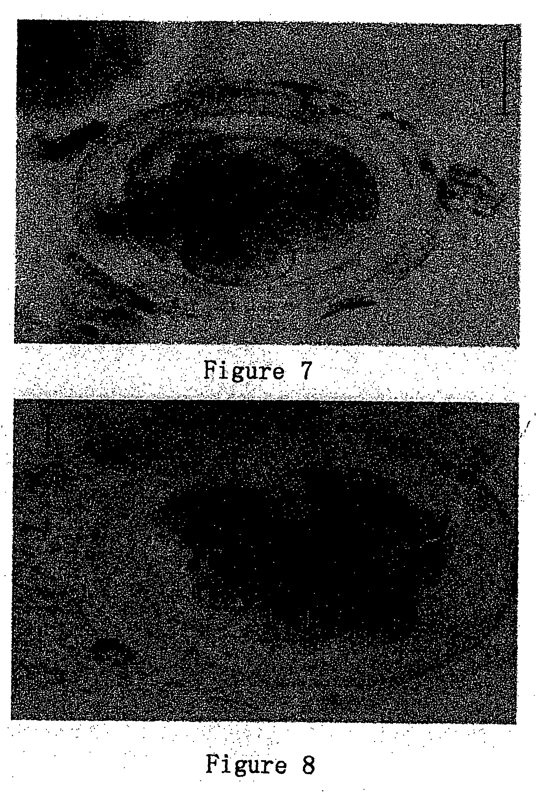

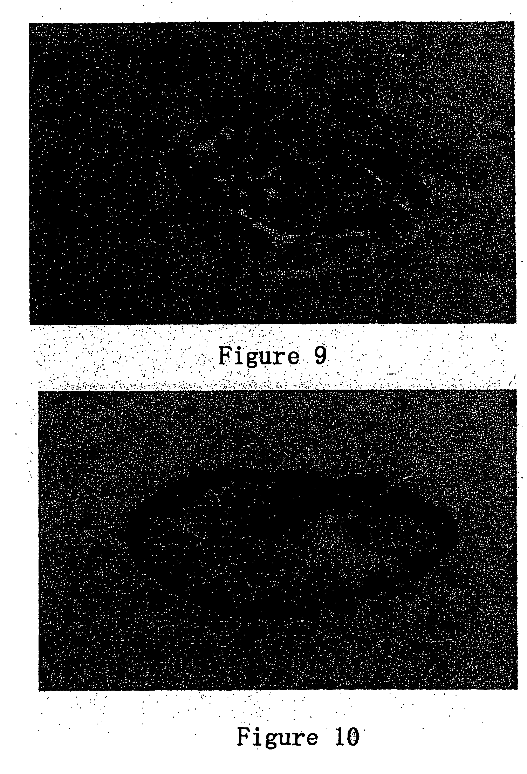

Oocytes were incubated in M2 solution with 7.5 µg/ml Cytochalasin B (Sigma) and enucleated by a needle with a bevel after maintained for 10 minutes at room temperature. After that, put the single donor fibroblast into the cytoplasm of the enucleated rabbit oocyte, forming a nt-unit. Many nt-units were cultured in RD medium, consisting of DMEM 42.5% (Gibco), RPMI-1640 42.5% (Gibco), 15% FBS (Hyclone). After 5-7 days, blastocysts were obtained (Figs. 7-10).

### Example 2

### Establishing Human nuclear transfer embryonic stem cells lines

Nt-units at the blastocyst stage were obtained as described above and pipetted up and down gently using a glass needle (stretched from a glass pipe of 3mm diameter) with a diameter smaller than the blastocyst to strip zona pellucid. Then, the inner cell masses of the blastocysts were isolated and plated onto feeder layers and cultured in 79% DMEM (Gibco), 20% FBS (Hyclone), 1% non-essential amino acid stock (Gibco), 0.1 mM β-mercaptoethanol (Gibco), 10 ng/ml LIF (R&D), 10 ng/ml bFGF (R&D) and 10µM Forskolin (Sigma) at 37°C in 5% CO₂, with half of the medium changed every 2 days.

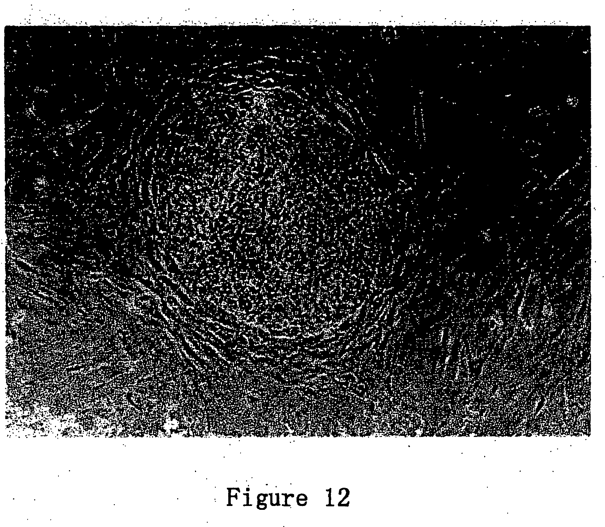

After 2-4 days' culturing, cell mass was observed to be growing on the feeder. After 7-20 days, colonies were observed (Fig. 12). The colonies were dispersed by enzyme or by mechanical means and passed onto a plate containing fresh feeder layer. After 20 passages, the ntES cells were cryopreserved.

### Fibroblast feeder layer

The feeder cells were derived from mouse embryos of 13-14 days old. After the removal of the head, liver, heart and esophagus of the embryos under sterile condition, the remains of the embryo were minced, digested in a pre-warmed solution containing 0.05% Trypsin/0.02% EDTA (Gibco), incubated at 37°C for 30 minutes. Remove the cell suspension and centrifuge at 1000 rpm for 5 minutes. The cells pellet was re-suspended in 90% DMEM (Gibco); 10% FBS (Hyclone); 50 IU penicillin-streptomycin (Gibco), 1% non-essential amino acid stock (Gibco) and 0.1 mM β-mercaptoethanol (Gibco), plated and incubated at 37°C, 5% CO₂. After passage 3 times, a feeder layer was treated with 10mM Mitomycin C (Sigma) for 3-4 hours and passaged on 4-well or 96-well plate. A feeder layer grew in a 37°C humidified incubator containing 5% CO₂ and used to prepare ntES Culture.

### Example 3

### Karyotyping

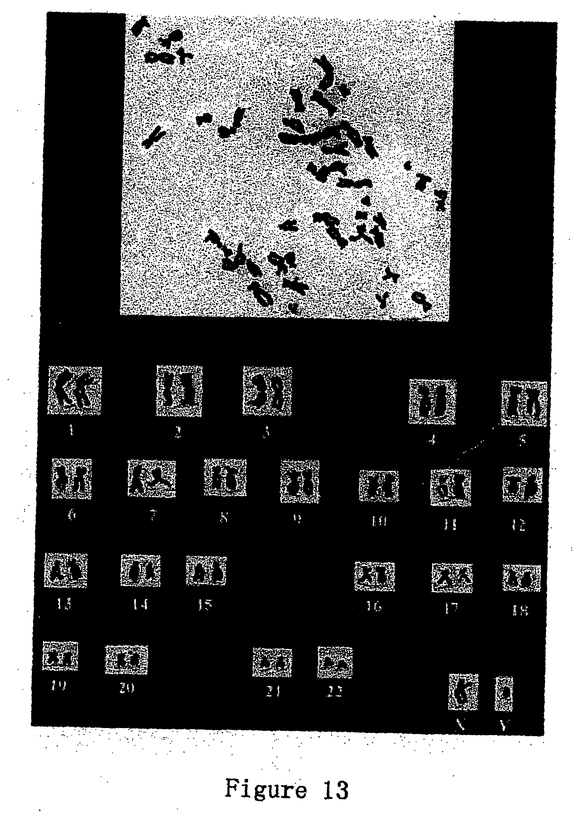

Put 10 µg/ml Colchicine in cell cultures. After 37°C for 4 hours, the cells were removed out from the plate and centrifuged cells at 1000 rpm for 8 minutes. Discard the supernatant, re-suspend cell pellet in pre-warmed 0.05 M KCI, and incubate at 37°C for 30 minutes. Centrifuge at 1000 rpm for 8 minutes, Discard the supernatant, re-suspend cell pellet in a fixation solution (methanol: ice acetic acid=3:1) and keep at room temperature for 15 minutes. Centrifuge at 1000 rpm for 8 minutes, Discard the supernatant, and re-suspend cells pellet again in a fixation solution at room temperature for 15 minutes. After centrifuging at 1000 rpm for 8 minutes, put cell pellet into a glass plate and stain with GIEMSA to examine the karyotype. One of he results is shown in figure 13.

While the present invention has been described with reference to the specific embodiments thereof, it should be understood by those skills in. the art that various changes and modification may be made without departing from the true spirit and scope of the invention. All such modifications are intended to be within the scope of the description and the claims appended hereto.

## Claims

1. A method of preparation the nuclear transfer unit, involving transplanting a donor somatic cell or cell nucleus into an enucleated oocyte of a species different from the nuclear donor and culturing the resultant nuclear transfer unit to a late pre-implantation stage under a proper condition.

2. The method of Claim 1, wherein the said the nuclear donor cell is a human cell, preferable an embryonic or adult somatic cell, and more preferable adult fibroblasts.

3. The method of Claim 1, wherein the oocyte is obtained from a mammalian or amphibian.

4. The method of Claim 3, wherein the oocyte is obtained from human.

5. The method of Claim 3, wherein the oocyte is obtained from leporid, preferable a rabbit.

6. The method of any one of Claims 3-5, wherein the oocyte is in the middle of divisional period, preferable in metaphase II stage.

7. The method of Claim 1, wherein the resultant nuclear transfer units are activated by being cultured at room temperature or by using activating agent.

8. The method of Claim 7, wherein the activating agent is selected from the group consisting of mannitol electrofusion solution, glucose electrofusion solution, sorbitol electrofusion solution and phosphate buffered solution, more preferably glucose buffer solution.

9. The method of Claim 1, wherein the activated nuclear transfer units are cultured in a medium selected from the group consisting of RD medium, M199 medium and DMEM medium, more preferable RD medium, to obtain the nuclear transfer units at various pre-implantation stages, including e.g. the 2∼4-cell, 8-cell, morula, blastocyst, and hatching blastocyst stages.

10. The method of Claim 1, wherein the activated nuclear transfer units are cultured in the medium selected from the group consisting of RD medium, M199 medium, and DMEM medium, and form co-culture system with the multiple cells style, e.g. granular cell, oviduct cells, STO (mouse fibroblast), to obtain the nuclear transfer units at various pre-implantation stages, including e.g. the 2∼4-cell, 8-cell, morula, blastocyst and hatching blastocyst stages.

11. Nuclear transfer units at all pre-implantation stages obtained according to the method of any one of Claims 1-10.

12. Nuclear transfer units at all pre-implantation stages obtained according to Claim 11, obtained by transplantation of human cells or cell nuclei into leporid enucleated oocytes.

13. Nuclear transfer units at all pre-implantation stages obtained according to Claim 11, obtained by transplantation of adult fibroblast cells or their nuclei into rabbits enucleated oocytes.

14. The use of nuclear transfer units at all pre-implantation stages obtained according to any one of Claims 11-13 in the preparation of human embryonic stem cells or embryonic stem-cells like cells or other types of embryo-derived stem cells.

15. The use of nuclear transfer units at all pre-implantation stages obtained according to any one of Claims 11-13 and cells derived therefrom in the treatment methods and commercial uses.
